# EUROPEAN PATENT SPECIFICATION

(11) **EP 1 485 160 B1**
(45) Date of publication and mention of the grant of the patent: **16.07.2008**
(21) Application number: 03728237.3
(22) Date of filing: 19.03.2003
(51) Int. Cl.: A61M 31/00

(54) **INTRADUCTAL MANAGEMENT OF BREAST LESIONS INVOLVING THERAPEUTIC OR DIAGNOSTIC AGENTS**
INTRADUKTALE BEHANDLUNG VON BRUSTLÄSIONEN UMFASSEND THERAPEUTISCHE UND DIAGNOSTISCHE MITTEL
GESTION INTRACANALAIRE DE LESIONS MAMMAIRES FAISANT INTERVENIR DES AGENTS THERAPEUTIQUES OU DIAGNOSTIQUES

(30) Priority: 19.03.2002 US 365168 P
(43) Date of publication of application: 15.12.2004
(73) Proprietor: CYTYC CORPORATION, Marlborough, MA 01752 (US)
(72) Inventor: HUNG, David, Redwood City, CA 94062 (US)
(74) Representative: Tombling, Adrian George
(86) International application number: PCT/US2003/007279
(87) International publication number: WO 2003/080171

(56) References cited:
- WO-A-00/51666
- WO-A-01/85219
- PECK RONALD A ET AL: "Phase I and pharmacokinetic study of the novel MDR1 and MRP1 inhibitor biricodar administered alone and in combination with doxorubicin." JOURNAL OF CLINICAL ONCOLOGY, vol. 19, no. 12, 15 June 2001 (2001-06-15), pages 3130-3141, XP009015753 ISSN: 0732-183X
- KABASAKAL L ET AL: "The effect of P-glycoprotein function inhibition with cyclosporine A on the biodistribution of Tc-99m sestamibi." CLINICAL NUCLEAR MEDICINE. UNITED STATES JAN 2000, vol. 25, no. 1, January 2000 (2000-01), pages 20-23, XP009015755 ISSN: 0363-9762

## Description

### TECHNICAL FIELD

The present invention relates to an apparatus for management of breast lesions and in particular enhancing imaging of breast tumors. A device according to the preamble of claim 1 is disclosed in WO-A-01/85219.

### BACKGROUND OF THE INVENTION

Breast cancer is a major cause of death in women. It is estimated that up to 10% of women in the United States are at risk of developing breast cancer in their lifetime. Methods of early detection have been developed such as physical examinations, regular self-examinations, mammography or tissue biopsy, however, inherent features of these methods limit their utility. Physical examinations and self-examinations may depend on the skill of the examiner and some lesions, particularly small-sized lesions, may be overlooked. Mammograms may sometimes be difficult to interpret in more dense breast tissue. Furthermore, mammograms may lack optimal sensitivity such that breast lesions may be present for many years and may develop to an advanced stage of disease before they are detectable on mammogram. Some breast tumors may grow undetected in breast tissue in excess of ten years before being detected by physical examination or mammography. Therefore, because advanced stage disease often carries a poor prognosis, reliance on mammogram may be less than optimal.

Tissue biopsy often requires a palpable lesion before sampling may be performed effectively at which time the lesion may have progressed to an advanced stage and may carry a poorer prognosis. If the lesion is more advanced, there may be a higher risk of treatment failure. This problem may be offset if the lesion could be detected earlier. Tissue biopsies may also cause tissue artifacts that may be problematic for proper diagnosis. For example, fine needle aspiration (FNA) may cause local reactions at the site of the biopsy such as scarring or inflammation that may make it difficult to visualize the tumor both grossly or microscopically. In such a situation, proper diagnosis is hampered and a tumor may be missed. Tissue biopsies may even cause spread of a tumor if the tumor or tumor cells are dislodged from the main tumor site into surrounding tissues. In addition, tissue biopsy is an invasive procedure that may cause patient discomfort and inconvenience. Therefore, effective non-invasive techniques in management of breast cancer is needed.

Nuclear medicine breast imaging has been used for visualizing or detecting breast lesions such as breast tumors. The process typically involves injecting a nuclear medicine imaging compound such as a radioactive tracer dye (i.e., Tc-99m sestamibi compound) into the bloodstream. The radioactive compound more actively accumulates in tumor cells than in normal (non-cancerous) cells so that a scan may then be performed to visualize "hot spots" corresponding to likely sites of tumor activity. Nuclear imaging has been shown to be especially effective in patients with dense breast tissue or in patients in whom a palpable breast mass is detected but cannot be imaged using other traditional techniques such as mammography or ultrasound. Further, nuclear imaging is useful when the presence of multi-focal tumor sites throughout the breast is suspected.

Typically administered intravenously, systemic administration of Tc-99m sestamibi exposes the patient to radioactive material throughout the body. Tc-99m sestamibi concentrates in tissue in proportion many factors including blood flow or metabolic activity. Tumors generally concentrate Tc-99m sestamibi to a greater extent because tumors tend to be more vascular than normal tissue and exhibit increased metabolic activity. Further, Tc-99m sestamibi concentration is enhanced in proportion to mitochondrial conentration and tumors may concentrate higher levels of Tc-99m sestamibi because tumors tend to have a higher intracellular mitochondrial concentration. Thus, the use of nuclear scanning with Tc-99m sestamibi is useful in detection of breast tumors.

However, although the dose of radiation from Tc-99m sestamibi in breast cancer diagnosis is low, there has been concern that the radioactive drug may result in long term complications associated with systemic radiation exposure as well as several other side effects associated with administration of Tc-99m sestamibi. Because Tc-99m sestamibi is administered systemically, there is general exposure to the compound and therefore increased risk of harmful side effects. For example, pregnant women need to be cautious when receiving even a small amount of exposure to radioactivity associated with the procedure because serious birth defects or other damage to the fetus may occur with pre-natal exposure to radiation. The impact of administration of Tc-99m sestamibi to elderly patients has not been fully elucidated. Therefore, it is possible that elderly patients may have unique side effects or problems with systemic administration of Tc-99m sestamibi and the resultant general exposure to radiation. It has been advised to take extra precaution with administering Tc-99m sestamibi systemically in elderly patients. Furthermore, numerous other side effects for all patients have been reported with systemic exposure to Tc-99m sestamibi, including a metallic taste, headache, flushing, nausea, vomiting, pruritus, rash, seizures, joint pains, shortness of breath, allergic reactions or bradycardia. Therefore, a need exists to minimize the side effects of systemic exposure to Tc-99m sestamibi.

Systemic administration of Tc-99m sestamibi may also not provide optimal imaging. Tc-99m sestamibi is typically administered through an indwelling intravenous catheter which allows the Tc-99m sestamibi to spread throughout the body through the bloodstream. Although there is some preferential uptake of Tc-99m sestamibi in tumor tissue as opposed to normal, non-cancerous tissue, there may still be confounding imaging data secondary to this generalized approach at administration of Tc-99m sestamibi. For example, there may be excessive uptake of the compound in normal lymph nodes. When other tissue takes up Tc-99m sestamibi, there may be obfuscation of any tumor tissue present and the diagnosis may be inadvertently missed. Attempts at minimizing this problem have been taken in the past including administering Tc-99m sestamibi via a vein in the contralateral arm. The contralateral arm (i.e., the arm opposite the suspected lesion) is chosen over the ipsilateral arm (i.e., the arm on the same side as the suspected lesion) in an attempt to avoid such confounding imaging data. However, this technique does not eliminate confounding imaging data altogether and, with systemic administration, there is often sufficient extraneous uptake of Tc-99m sestamibi to confound results despite this attempt at minimizing the problem.

In addition, nuclear imaging with Tc-99m sestamibi compound may be further limited by molecular "drug pumps" that expel the sestamibi compound from tumor cells. A protein, P-glycoprotein (PGP), may function as such a molecular "drug pump" to pump sestamibi compound from tumor cells. This causes decreased uptake of Tc-99m sestamibi. Because the sensitivity of the nuclear scan depends on the presence of Tc-99m sestamibi in the tumor cells, expelling of Tc-99m sestamibi from tumor cells by PGP results in degradation of the nuclear scan image. If tumor cells are present, the sestamibi compound may be expelled from the tumor cells via the PGP "pumps" and therefore may not be adequately visualized.

For the foregoing reasons, the systemic administration of Tc-99m sestamibi in the detection and management of breast cancer is limited. Side effects and teratogenic effects of Tc-99m sestamibi make systemic administration problematic. Systemic administration may also not provide optimal concentration of Tc-99m sestamibi in the breast tissue and Tc-99m sestamibi taken up by tumor cells in the breast may be expelled by PGP "drug pumps" resulting in degradation of the nuclear scan image.

Thus, there exists a need for a method of effectively performing nuclear imaging of breast tumors via administering nuclear scintigraphy agents such as Tc-99m sestamibi such that radiation exposure is limited, side effects are minimized and imaging of tumor cells is not impaired.

### SUMMARY OF THE INVENTION

The present invention relates to an apparatus for administering a radioactive tracer dye into a breast duct of the breast and administering an inhibitor compound into the breast duct.

Nuclear scan imaging of breast tumors may be performed after successful administration and tumor cell uptake of Tc-99m sestamibi. The present invention relates to systems for administering Tc-99m sestamibi for nuclear scanning directly into a breast duct system. Direct and local administration of Tc-99m sestamibi into the breast duct system as opposed to systemic administration of Tc-99m sestamibi permits improved delivery of Tc-99m sestamibi at lower doses to the breast tissue while avoiding many unwanted side effects associated with systemic administration of Tc-99m sestamibi such as metallic taste, headache, nausea, vomiting, diarrhea, pruritis, or seizures, for example.

The present invention further relates to tumor cell retention of Tc-99m sestamibi for improved nuclear scanning. Tc-99m sestamibi is expelled from tumor cells by a MultiDrug Resistance Gene (MDR gene) product, P-glycoprotein (PGP). PGP functions to expel Tc-99m sestamibi from tumor cells thereby decreasing the effectiveness of Tc-99m sestamibi and consequently decreasing the ability to image the tumor on nuclear scans. PGP also expels certain chemotherapeutic agents useful in the treatment of cancer from tumor cells causing "resistance" to those chemotherapeutic agents. The present invention provides a system for direct and local administration of inhibitor compounds that inhibit PGP into a breast duct system (including a single duct or a plurality of ducts) such that Tc-99m sestamibi is not substantially expelled from the breast tumor cells by PGP and nuclear scan imaging of the breast tumor may be effectively performed. Likewise, the inhibitor compounds inhibit the expulsion of chemotherapeutic agents from tumor cells thereby reversing the "resistance" to those chemotherapeutic agents by tumor cells.

### BRIEF DESCRIPTION OF THE DRAWINGS

Fig. 1 illustrates an exemplary apparatus for administering compounds into breast tissue.

Fig. 2 illustrates an exemplary apparatus for administering compounds into a breast duct system involving a single port.

Fig. 3. illustrates an exemplary apparatus for administering compounds into a breast duct system involving Y-tube-shaped catheter.

### DETAILED DESCRIPTION OF THE INVENTION

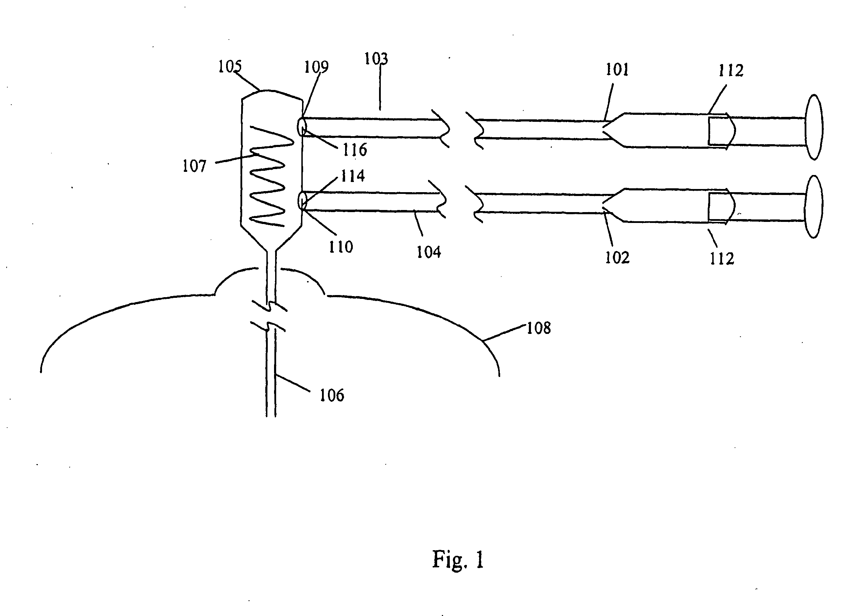

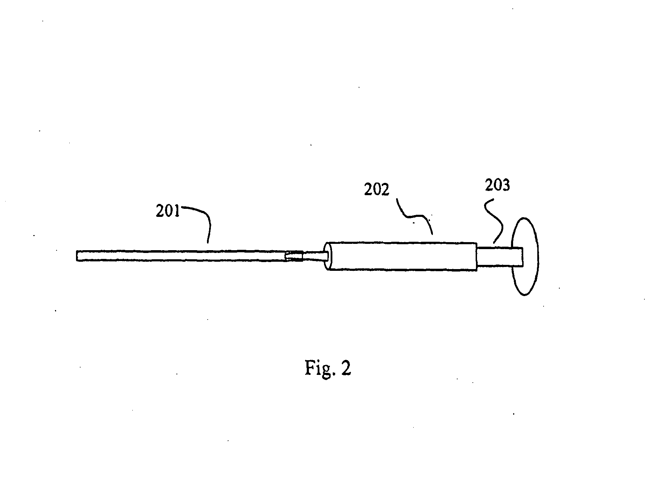

The present invention provides an apparatus for administering diagnostic and/or therapeutic agents such as nuclear scintigraphy agents in the management of breast lesions such as breast cancer. The apparatus of the present invention may be introduced into a breast duct and diagnostic and/or therapeutic agents may be locally introduced into the breast duct. Breast lesions may be identified early in their formation even before they are grossly visible or palpable. Fig. 1 illustrates an exemplary embodiment of an apparatus for administering nuclear scintigraphy agents for management of breast lesions in which multiple ports are utilized to introduce material into a breast duct. Fig. 2 illustrates an alternative embodiment of a device for administering agents in which a single port is utilized to introduce material into a breast duct. It should be noted that the illustrated devices are for illustration purposes only and they are not meant to limit the present invention as many similar devices may be utilized by a skilled artisan without departing from the scope of the invention.

The apparatus of the present invention may be introduced into a breast duct through a ductal opening in the nipple and nuclear scintigraphy agents may be locally introduced into the breast duct. Thus, without the need for percutaneous introduction of material, breast lesions may be identified early in their formation even before they are grossly visible or palpable.

As exemplified in Fig. 1, which demonstrates an illustrative embodiment of an apparatus 100 of the present invention, the exemplary apparatus for introducing material into a breast duct system and/or obtaining a biological sample from a breast duct contains a ductal access device such as a catheter or cannula that may be inserted into the breast duct. Material may be introduced into the breast duct system and/or a biological sample may be obtained from the breast duct system via this single ductal access device. As a result, ductal lavage may be performed on one or more ducts and material may be introduced into the duct(s) via the apparatus 100. The apparatus 100 may thus be placed in a breast duct or plurality of breast ducts and utilized for ductal lavage and introduction of material in a single duct or a plurality of ducts without removing the apparatus 100 from the breast duct or plurality of breast ducts. Alternatively, separate catheters or cannulas can be used to perform these tasks. U.S. Patent Application Serial Number 09/473,510, David Hung, et al., filed December 28, 1999 discloses an exemplary apparatus having an elongated ductal access device that can be used with the present invention for positioning within a breast duct.

Fig. 1 illustrates one exemplary embodiment of the apparatus 100 of the present invention. The apparatus 100 contains a ductal access device such as a cannula or a catheter 106 for positioning within a breast duct and a main chamber or manifold 105 in fluid communication with the catheter 106. The ductal access device such as the catheter 106 can have an internal lumen extending between its ends. The main chamber 105 has an internal volume and an internal diameter that is greater than that of the catheter 106. The main chamber 105 also includes a first port 110 and a second port 109. These ports 109, 110 can be placed at any position discussed in U.S. Patent Application No. 09/473,510. For example, the second port 109 can be placed at the terminal end of the main chamber 105 and inline with the catheter 106. Additionally, the first port 110 can be positioned as close to the catheter 106 as possible. Moreover, these ports 109, 110 can be vertically aligned with each other along the wall of the main chamber 105 or offset around the circumference of the main chamber 105.

Fluids and other materials can be introduced into and removed from the main chamber 105 through either of the illustrated ports 109, 110. As illustrated in Fig. 1, the first port 110 is connected to a first conduit 104 that has a port 102 for receiving an instrument such as a syringe 112. The second port 109 is connected to a second conduit 103 that has a port 101 for receiving a syringe 112. The syringes 112 can be replaced by any known collection and/or infusion device.

As discussed above, the port 102 and conduit 104 can be used to infuse a fluid into the main chamber 105 and into the duct via the catheter 106. In this case, material or fluid is placed into the first port 102 and positive pressure is exerted at the first port 102 to expel the material or fluid into the main chamber 105 and into the breast duct system via the catheter 106. Alternatively, material or fluid may be placed into the second port 101 and expelled into the main chamber 105 through the second conduit 103 by exerting positive pressure at the second port 101. The material or fluid may thus be administered into the chosen breast duct or ducts.

As previously mentioned, the port 101 and the conduit 103 can be used to collect material received from the duct and contained in the main chamber 105. For example, negative pressure may be exerted at the first port 109 by the operation of the syringe 112 connected to the port 101. This action produces a negative pressure in the main chamber 105 and draws the material obtained from the breast duct and residing in the main chamber 105 into the conduit 103 and the syringe 112 or other collection device.

The above descriptions of which ports and conduits are used to introduce material or fluid into the duct and collect material from within the duct are merely exemplary. Either set of conduits and ports can be used to perform either of these functions. Extraction of biological material which can include ductal fluids, cells (cell clumps) and the ductal wash fluid from the breast duct system may be accomplished by externally massaging the breast after the ductal wash fluid has been introduced into the duct. Additionally, negative pressure within the main chamber 105 can be caused by the operation of one or more of these syringes 112.

As shown in the exemplary embodiment of Fig. 1, valves 114, 116 may regulate the flow of material or fluid into and out of the main chamber 105 through the input port 110 and output port 109, respectively. The catheter 106 may have an internal lumen of a diameter sufficiently sized such that insertion into a breast duct system is facilitated while permitting the passage of desired agents and material. The catheter 106 may, for example, have a lumen diameter of 0.007 inches (or 0.178 mm) or greater, or a lumen diameter in the range from 0.007 inches (or 0.178 mm) to 0.047 inches (or 1.19 mm). Further, the catheter 106 may contain indicia on its surface to indicate the depth of insertion such that a user may be fully aware of the depth of insertion of the catheter 106 during insertion of the catheter 106 into the breast duct. Further, the catheter 106 or other part of apparatus 100 may contain a safety mechanism such as a stop element (not shown) such that the catheter 106 may not be further advanced into the breast duct system after a certain depth is attained. Such a stop element may be variously designed but may comprise, for example, a collar affixed to or formed on an exterior surface of the catheter 106, the collar being of a width greater than the diameter of the catheter 106.

Fig. 2 illustrates another exemplary embodiment of an apparatus for administering agents into a breast duct system. In this embodiment, the apparatus is a single lumen device comprising a catheter 201 in connection with a syringe 202. The syringe 202 enables introduction of the below discussed desired agents into the breast duct system. A plunger 203 may be situated at a top end of the syringe 202, for example, and may be used to introduce agents contained within the syringe 202 into the breast duct system (not shown).

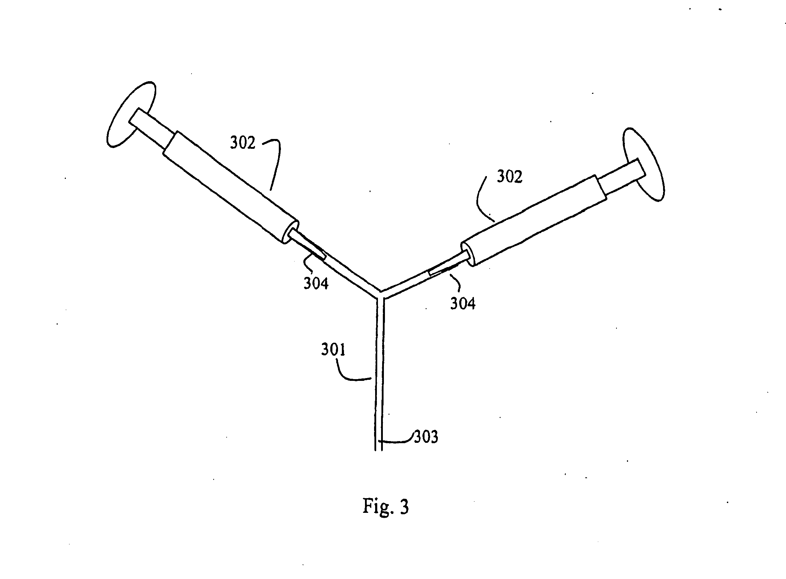

Fig. 3 illustrates another exemplary embodiment of an apparatus for administering agents into a breast duct system. In this embodiment, a syringe 302 is connected to a Y-tube-shaped catheter 301 at each of a plurality of proximal ends 304 of which two are shown. The distal end 303 of the Y-tube-shaped catheter 301 may be inserted into a breast duct system through a ductal opening in a nipple surface. Desired agents may be introduced into the breast duct system from any of the plurality of proximal ends 304 of the Y-tube-shaped catheter 301. This exemplary embodiment allows multiple agents to be administered separately or allows the mixing of separately administered agents.

It will be appreciated that the disclosed exemplary embodiments of an apparatus for administering agents into a breast duct system are for illustration purposes only and are not intended to limit the present invention. Any suitable device suitable for injecting or infusing fluid into a duct may be utilized for introducing desired agents into a breast duct system may be employed without deviating from the scope of the present invention.

In an exemplary embodiment of the present invention, Tc-99m sestamibi is introduced into a breast duct for radiographic management of intraductal breast lesions. Tc-99m sestamibi is a radioactive tracer dye that may accumulate in tumor cells and is approved for breast imaging in the United States in nuclear medicine breast imaging. Typically, Tc-99m sestamibi is administered systemically and preferentially taken up by tumor cells. As discussed above, systemic administration of Tc-99m sestamibi may result in unwanted side effects including metallic taste, headache, nausea, vomiting, pruritis, or seizures, for example. Also, systemic administration may not permit efficient concentration at the anatomic site of interest. Thus, in this example, improved Tc-99m sestamibi levels in the breast is achieved by administering Tc-99m sestamibi directly into a breast duct system such that there is enhanced concentration of Tc-99m sestamibi in the breast with virtually no systemic radiation exposure and minimal side effects. As an example, Tc-99m sestamibi is introduced directly into a breast duct system through a ductal orifice in a breast nipple surface via the catheter 106. Tc-99m sestamibi is introduced via the first port 102, for example, and passes through the first conduit 104 and into the main chamber 105. From the main chamber 105, Tc-99m sestamibi passes through the catheter 106 and into the breast duct system. The Tc-99m sestamibi compound thus introduced may be taken up by tumor cells present in the breast. A gamma camera capable of detecting the radioactive tracer dye is used to image the breast to detect uptake of the dye. Detection of "hot spots" may indicate the presence of tumor.

This exemplary embodiment demonstrates the introduction of Tc-99m sestamibi into a breast duct system for diagnosis of breast cancer while not only minimizing the general whole-body exposure to radiation from Tc-99m sestamibi thereby protecting the patient from radiation or teratogenic effects, but also enhancing the concentration of Tc-99m sestamibi in the breast which can provide improved nuclear scanning images. In this way, side effects such as metallic taste, headache, nausea, vomiting, diarrhea, pruritis, rash, or seizures, for example, may be avoided. In addition, local and direct administration of Tc-99m sestamibi results in improved delivery to the anatomic site where it is desired rather than a systemic administration of Tc-99m sestamibi which must travel through the bloodstream and is therefore not as well concentrated or directed as the direct administration technique of the present invention. This may result in a lower required dose of Tc-99m sestamibi and thus lower toxicity levels as well.

Introduction of Tc-99m sestamibi into a breast duct system for management of breast tumor such that Tc-99m sestamibi may be taken up by tumor cells to enable visualization on nuclear scanning may be further hampered by certain cellular proteins. Tumor cells contain a "drug pump" which is made of a protein product of the Multidrug Resistance gene (MDR gene), P-glycoprotein (PGP). PGP pumps Tc-99m sestamibi out of tumor cells thereby interfering with proper visualization of breast tumor on nuclear scans. PGP also pumps chemotherapeutic agents out of tumor cells as well resulting in decreased effectiveness of these chemotherapeutic agents in the treatment of cancer. As a result, the tumor cells containing the MDR gene product become resistant to the chemotherapeutic agents that are pumped out of the cell just as they become less visible on nuclear scanning as a result of Tc-99m sestamibi being pumped out of tumor cells. Thus, PGP facilitates resistance in tumor cells by pumping certain compounds out of tumor cells. This results in decreased intracellular effect of these compounds. PGP pumps certain cancer chemotherapeutic agents out of tumor cells thereby decreasing the efficacy of those chemotherapeutic agents and also may expel Tc-99m sestamibi from tumor cells leading to tumor cell resistance to chemotherapeutic agents and degradation of nuclear scans. Inhibitor compounds that block the PGP protein pump have been developed with the goal of preventing the expulsion of agents from tumor cells via the PGP pump. If the inhibitor compound is administered systemically in conjunction with the chemotherapeutic agents, the chemotherapeutic agents may accumulate in the tumor cells because the inhibitor compound simultaneously blocks the PGP pump mechanism that would serve to clear the chemotherapeutic agents. Thus, the tumor cells regain their sensitivity to the chemotherapeutic agents.

Uptake of Tc-99m sestamibi provides for proper visualization of tumor in nuclear scans as described, however, when PGP expels Tc-99m sestamibi, tumor cells may not accumulate a sufficient level of Tc-99m sestamibi to light up on nuclear scan. If the Tc-99m sestamibi does not accumulate sufficiently in tumor cells, there may be decreased sensitivity for detection of intraductal lesions. Because the inhibitor compounds may inhibit the PGP protein pump, Tc-99m sestamibi may be administered in conjunction with the inhibitor compound to increase the efficacy of the Tc-99m sestamibi in a similar manner of increasing the efficacy of chemotherapeutic agents by administering the inhibitor compound.

There are several inhibitor compounds that may be used to block the action of the PGP protein pump to prevent the expulsion of chemotherapeutic agents and Tc-99m sestamibi from tumor cells. This results in increased effectiveness of chemotherapeutic agents to kill tumor cells and also provides better visualization of the breast tumor on nuclear scanning with Tc-99m sestamibi. The inhibitor compounds include, for example, biricodar dicitrate or VX-853. Typically, biricodar dicitrate is administered systemically in conjunction with chemotherapeutic agents enhancing retention of the chemotherapeutic agent in tumor cells and thus enhancing killing of the tumor. However, such inhibitor compounds, such as biricodar dicitrate, may also be administered in conjunction with Tc-99m sestabmibi to reduce expulsion of Tc-99m sestamibi from tumor cells during nuclear scanning. Furthermore, to decrease the incidence and severity of side effects associated with generalized exposure to the inhibitor compounds as well as improved delivery of the compounds to the breast, the inhibitor compound may be administered locally directly into the breast duct system through a breast nipple surface.

The inhibitor compounds may be administered by the exemplary apparatus 100 illustrated in Fig. 1. The inhibitor compound may be contained in a first port 102 of the apparatus of Fig. 1 and the catheter 106 is placed in the breast duct system through the breast nipple surface. Pressure is exerted at the first port 102 causing the inhibitor compound contained therein to pass through the first conduit 104 and into the main chamber 105. From the main chamber 105, continued increased pressure causes the inhibitor compound to continue through the catheter 106 and into the breast duct system. Alternatively, the inhibitor compound may be contained in the second port 101 and may pass through the second conduit 103 to enter the main chamber 105. After the inhibitor compound enters the breast duct system via the catheter 106, breast tumor cells, if present, take up the inhibitor compound which inhibits the MDR gene product, PGP, in the tumor cells. Inhibition of PGP results in greater chemotherapeutic efficiency and reversal of "resistance" to certain chemotherapeutic agents that are susceptible to being expelled from tumor cells through the actions of PGP as well as enhanced nuclear scanning imaging from Tc-99m sestamibi since Tc-99m sestamibi is also susceptible to being expelled from tumor cells through the actions of PGP.

The apparatus illustrated in Fig. 1 may also be used to introduce both Tc-99m sestamibi and the inhibitor compound either simultaneously or sequentially. For example, after the catheter 106 is situated in the breast duct system, Tc-99m sestamibi may be placed in the first port 102 and the inhibitor compound may be placed in the second pot 101. Pressure is exerted at both the first port 102 and the second port 101 causing both compounds, Tc-99m sestamibi and the inhibitor compound, to pass through the first conduit 104 and second conduit 103, respectively and into the main chamber 105. Continued increase pressure exerted at both the first port 102 and the second port 101 causes the resultant mixture of Tc-99m sestamibi and the inhibitor compound to pass through the catheter 106 and into the breast duct system. Once in the breast duct system, Tc-99m sestamibi may be taken up by breast tumor cells and nuclear scanning will produce a nuclear scan image. Also, the inhibitor compound prevents expulsion of Tc-99m sestamibi from the tumor cells and thus enhances the nuclear scanning imaging process.

The apparatus illustrated in Fig. 1 may also be used to administer Tc-99m sestamibi and inhibitor compounds sequentially. For example, Tc-99m sestamibi may be introduced into the breast duct system from the first port 102 via the catheter 106 in the breast duct system as described. After Tc-99m sestamibi has been administered into the breast duct system, the inhibitor compound may be introduced into the breast duct system from either the first port 102 or the second port 101 through the catheter 106. Likewise, the inhibitor compound may be introduced first from either the first port 102 or the second port 101 followed by administration of Tc-99m sestamibi from either the first port 102 or the second port 101. It should be noted that this is only intended to illustrate the general principle of administration of the desired compounds and should not be construed as limiting in any way. For example, any of the compounds may be administered from either the first port 102 or the second port 101. Also, there may be any number of ports on the apparatus 100.

Fig. 2 illustrates another exemplary apparatus that may be used in introducing material into a breast duct system. For example, Tc-99m sestamibi may be contained in the syringe 202 and the catheter 201 may be positioned in the breast duct system of a breast through the breast nipple surface. Pressure is exerted at the top end of the plunger 203 such that the pressure is transmitted through to the lumen of the syringe 202 where the Tc-99m sestamibi is contained. The Tc-99m sestamibi thus passes through the catheter 201 and into the breast duct system. Once in the breast duct system, Tc-99m sestamibi may be taken up by tumor cells present therein thus permitting the attainment of nuclear imaging scans of the breast tumor. The apparatus of Fig. 2 may also be used to introduce the inhibitor compound into the breast duct system. Because Tc-99m sestamibi may be expelled from tumor cells in the breast due to action of the MDR gene product, PGP, as described, an inhibitor compound that inhibits the action of PGP may be administered by the exemplary apparatus illustrated in Fig. 2. The inhibitor compound is placed in the lumen of the syringe 202 and the catheter 201, which is connected to the bottom end of the syringe, is placed into the breast duct system. The catheter 201 may contain markings on its surface such that the depth of insertion may be readily ascertainable. The depth of insertion may vary based on the unique qualities and complexity of a given breast duct system. One of skill in the art would readily determine the proper depth during examination of the breast duct system while inserting the catheter 201. After the catheter 201 is in the breast duct system at the desired depth, pressure is exerted at the top end of the plunger 203. This downward pressure causes the pressure to increase within the lumen of the syringe 202 and subsequent expulsion of the inhibitor compound within the lumen of the syringe 202 into the breast duct system via the catheter 201. As a result, the inhibitor compound enters the breast duct system and may be taken up by tumor cells in the breast. After the breast tumor cells take up the inhibitor compound, the inhibitor compound inhibits the functioning of the MDR gene product, PGP, within the tumor cells, thus preventing rapid expulsion of Tc-99m sestamibi from the tumor cells. It should be noted that the invention is not so limited as administration of Tc-99m sestamibi and the inhibitor compound may be in any order or even simultaneously. For example, the inhibitor compound may be introduced into the breast duct system first followed by the administration of Tc-99m sestamibi or both the inhibitor compound and Tc-99m sestamibi may be placed into the lumen of the syringe 202. In this embodiment, the catheter 201 is placed into the breast duct system and pressure is exerted at the top end of the plunger 203. This pressure causes increased pressure within the lumen of the syringe 202 which contains the mixture of inhibitor compound and Tc-99m sestamibi. The inhibitor compound/ Tc-99m sestamibi mixture is thus passed into the breast duct system via the catheter 201 where the compounds may be taken up by breast tumor cells.

Fig. 3 illustrates another exemplary apparatus for administering compounds in the present invention The Y-tube-shaped catheter 301 comprises a plurality of ends. A distal end of the Y-Tube-Shaped catheter 301 device may be positioned into a breast duct system and the proximal ends may contain devices for administering compounds such as syringes 302. There may be any number of syringes corresponding to any number of branches of the Y-tube-shaped catheter 301 of which two are illustrated in Fig. 3. As an example, the distal end of the y-tube-shaped catheter 301 device is placed into a breast duct system. One proximal end of the catheter 301 contains a syringe 302 containing Tc-99m sestamibi and another proximal end of the catheter 301 contains a syringe 302 containing an inhibitor compound that inhibits the action of the MDR gene product, PGP, in tumor cells. Pressure is exerted at both syringes 302 causing Tc-99m sestamibi and the inhibitor compounds to enter the Y-tube-shaped catheter 301 from their respective syringes 302. The inhibitor compound and Tc-99m sestamibi passes through the Y-tube-shaped catheter 301 and into the breast duct system where both the inhibitor compound and the Tc-99m sestamibi are taken up by tumor cells. The inhibitor compound inhibits the action of PGP in expelling the Tc-99m sestamibi from the tumor cells thereby enhancing the nuclear imaging capabilities of the breast tumor by Tc-99m sestamibi. Alternatively, the inhibitor compound and the Tc-99m sestamibi may be administered sequentially in any order. In any case, both compounds are taken up by the tumor cells for effective nuclear imaging of breast tumor.

In each of these embodiments, Tc-99m sestamibi is administered directly into the breast duct system through the ductal opening(s)in the breast nipple surface. Such an administration of Tc-99m sestamibi achieves improved delivery of Tc-99m sestamibi to breast tumor cells at lower doses with consequent avoidance of unwanted side effects of systemic administration of Tc-99m sestamibi including metallic taste, headache, nausea, vomiting, diarrhea, seizures, or teratogenic effects. To offset the action of PGP (the gene product of the MDR gene) that pumps Tc-99m sestamibi (as well as certain chemotherapeutic agents) out of tumor cells thereby decreasing the effectiveness of these compounds, inhibitor compounds such as biricodar dicitrate that are effective in inhibiting PGP in tumor cells may also be administered by any of the embodiments described herein. Local and direct administration of the inhibitor compounds into the breast duct(s) also results in improved delivery of the compounds to breast tumor cells at lower doses with consequent avoidance of unwanted side effects associated with systemic administration of the inhibitor compounds. The inhibitor compounds may be administered either simultaneously or sequentially with Tc-99m sestamibi.

Because sestamibi compounds may be pumped out of tumor cells through PGP pumps as described above, sensitivity of tumor detection may be improved if Tc-99m sestamibi is administered in conjunction with the inhibitor compound. Administration of an inhibitor compound may effectively block PGP pumps and may result in improved accumulation of Tc-99m sestamibi in tumor cells which may in turn result in increased sensitivity to visualization of breast tumors on nuclear scan. Further, both the sestamibi compound and the inhibitor compound may be administered locally rather than systemically which may result in decreased side effects or toxicity of the compounds.

Although the illustrative embodiments of the invention have been described, a wide range of modifications, changes and substitutions is intended in the foregoing disclosure. It is understood that the present invention can take many forms and embodiments. The embodiments shown herein are intended to illustrate rather than to limit the invention, it being appreciated that variations may be made without departing from the scope of the invention.

## Claims

1. An apparatus (100) for administering an agent into a mammary gland duct comprising:
a catheter (106) capable of being inserted into a mammary gland duct;
a first chamber (102) containing a radioactive tracer dye; **characterized by**
a second chamber (101) containing an inhibitor compound, wherein the inhibitor compound inhibits a cellular pump; and
an infusion device (112) for administering the contents of the first and second chambers into the mammary gland duct,
wherein the first and second chambers are in fluid communication with the catheter.

2. The apparatus (100) of claim 1, wherein separate infusion devices (112) are used for administration of the contents of the first (102) and second (101) chambers.

3. The apparatus (100) of claim 1 wherein the radioactive tracer dye is Tc-99m sestamibi compound.

4. The apparatus (100) of claim 1, which enables the inhibitor compound and the radioactive tracer dye to be administered into the breast duct from the chambers (101, 102) simultaneously.

5. The apparatus (100) of claim 1, which enables the inhibitor compound and the radioactive tracer dye to be administered into the breast duct from the chambers (101, 102) sequentially.

6. The apparatus (100) of claim 1, wherein the inhibitor compound comprises biricodar dicitrate.

7. The apparatus (100) of claim 1, wherein the cellular pump is P-glycoprotein (PGP).

## Patentansprüche

1. Gerät (100) zum Verabreichen eines Mittels in einen Brustdrüsengang umfassend:
einen Katheter (106), der in der Lage ist, in einen Brustdrüsengang eingeführt zu werden;
eine erste Kammer (102), die einen radioaktiven Tracer-Farbstoff enthält; **gekennzeichnet durch**
eine zweite Kammer (101), die eine Inhibitorverbindung enthält, wobei die Inhibitorverbindung eine zelluläre Pumpe inhibiert; und
eine Infusionsvorrichtung (112) zum Verabreichen der Inhalte der ersten und zweiten Kammer in den Brustdrüsengang,
wobei die erste und zweite Kammer in Flüssigkeitsaustausch mit dem Katheter stehen.

2. Gerät (100) nach Anspruch 1, wobei getrennte Infusionsvorrichtungen (112) zur Verabreichung der Inhalte der ersten (102) und zweiten (101) Kammer verwendet werden.

3. Gerät (100) nach Anspruch 1, wobei der radioaktive Tracer-Farbstoff eine Tc-99m Sestamibi Verbindung ist.

4. Gerät (100) nach Anspruch 1, das ermöglicht, dass die Inhibitorverbindung und der radioaktive Tracer-Farbstoff in den Brustgang aus den Kammern (101, 102) gleichzeitig verabreicht werden.

5. Gerät (100) nach Anspruch 1, das ermöglicht, dass die Inhibitorverbindung und der radioaktive Tracer-Farbstoff in den Brustgang aus den Kammern (101, 102) nacheinander verabreicht werden.

6. Gerät (100) nach Anspruch 1, wobei die Inhibitorverbindung Biricodar Dicitrat umfasst.

7. Gerät (100) nach Anspruch 1, wobei die zelluläre Pumpe P-Glycoprotein (PGP) ist.

## Revendications

1. Appareil (100) pour administrer un agent dans un canal de glande mammaire comprenant :
un cathéter (106) capable d'être inséré dans un canal de glande mammaire ;
une première chambre (102) contenant un colorant traceur radioactif ; **caractérisé par**
une deuxième chambre (101) contenant un composé inhibiteur, dans laquelle le composé inhibiteur inhibe une pompe cellulaire ; et
un dispositif de perfusion (112) pour administrer le contenu des première et deuxième chambres dans le canal de la glande mammaire,
dans lequel les première et deuxième chambres sont en communication par un fluide avec le cathéter.

2. Appareil (100) selon la revendication 1, dans lequel des dispositifs de perfusion distincts (112) sont utilisés pour l'administration du contenu des première (102) et deuxième (101) chambres.

3. Appareil (100) selon la revendication 1, dans lequel le colorant traceur radioactif est un composé sestamibi Tc-99m.

4. Appareil (100) selon la revendication 1, qui permet au composé inhibiteur et au colorant traceur radioactif d'être administrés dans le canal mammaire depuis les chambres (101, 102) de manière simultanée.

5. Appareil (100) selon la revendication 1, qui permet au composé inhibiteur et au colorant traceur radioactif d'être administrés dans le canal mammaire depuis les chambres (101, 102) de manière séquentielle.

6. Appareil (100) selon la revendication 1, dans lequel le composé inhibiteur comprend du dicitrate de biricodar.

7. Appareil (100) selon la revendication 1, dans lequel la pompe cellulaire est la glycoprotéine P (PGP).
